# EUROPÄISCHE PATENTSCHRIFT

(11) **EP 2 324 781 B1**
(45) Veröffentlichungstag und Bekanntmachung des Hinweises auf die Patenterteilung: **09.01.2013**
(21) Anmeldenummer: 09014509.5
(22) Anmeldetag: 20.11.2009
(51) Int. Cl.: A61B 17/17

(54) **Chirurgisches Führungsinstrument zum Bearbeiten von Facettengelenkfortsätzen an Wirbelkörpern**
Surgical guide instrument for working on facet joint extensions in spines
Instrument chirurgical de guidage de traitement de prolongements d'articulations de facettes sur des corps vertébraux

(43) Veröffentlichungstag der Anmeldung: 25.05.2011
(73) Patentinhaber: FACET-LINK Inc., Rockaway NJ 07866 (US)
(72) Erfinder: Link, Helmut D., 22397 Hamburg (DE)
(74) Vertreter: Glawe, Delfs, Moll

(56) Entgegenhaltungen:
- EP-A1- 1 442 714
- WO-A1-93/22975
- US-A- 2 181 746

## Beschreibung

Die Erfindung betrifft ein chirurgisches Führungsinstrument für die Bearbeitung von Facettengelenkfortsätzen an Wirbelkörpern.

Es sind Prothesen zum Ersatz von Gelenkflächen von Facettengelenken bekannt, bei denen ein Schaft der Prothese in einer Ausnehmung des Facettengelenkfortsatzes aufgenommen wird, siehe EP 1 959 871. Solche Ausnehmungen in den Knochen werden normalerweise mit einem Fräser erzeugt. Bislang wird der Fräser vom Chirurgen freihändig geführt. Allerdings befinden sich in der Nähe des Operationsgebiets bedeutende Nerven- und Blutbahnen. Eine unbedachte Bewegung des Chirurgen kann dazu führen, dass diese verletzt werden.

Eine chirurgische Zange mit den Merkmalen des Oberbegriffs des Anspruchs 1 ist aus der US 2,181,746 bekannt.

Der Erfindung liegt die Aufgabe zu Grunde, ein Führungsinstrument für die Bearbeitung von Facettengelenkfortsätzen vorzustellen, mit denen das Risiko einer unbeabsichtigten Verletzung des Patienten vermindert wird. Die Aufgabe wird gelöst durch die Merkmale der unabhängigen Ansprüche. Vorteilhafte Ausführungsformen finden sich in den Unteransprüchen.

Bei dem chirurgischen Führungsinstrument sind erfindungsgemäß zwei Zangenköpfe an einem distalen Ende ausgebildet, die für eine Zangenbewegung relativ zueinander ausgelegt sind. Das Führungsinstrument umfasst eine Führungsschiene, die eine Führungsachse für einen Fräser definiert. Die Führungsschiene ist um eine erste Schwenkachse schwenkbar, so dass die Führungsachse mit der Schwenkbewegung um die erste Schwenkachse einen Arbeitssektor überspannt und dass der Arbeitssektor eine gedachte Verbindungslinie zwischen den Zangenköpfen schneidet.

Der Erfindungsgedanke besteht darin, dass mit den Zangenköpfen an dem zu bearbeitenden Facettengelenkfortsatz angegriffen wird, um das Führungsinstrument so in eine definierte Stellung relativ zu dem Facettengelenkfortsatz zu bringen. Die Führungsschiene ist damit ebenfalls in einer definierten Stellung relativ zu dem Facettengelenkfortsatz und die Bewegungsfreiheit der Führungsschiene ist auf Schwenkbewegungen relativ zu dem Führungsinstrument beschränkt. Insbesondere überstreicht die Führungsachse der Führungsschiene bei einer Schwenkbewegung um die erste Schwenkachse einen Arbeitssektor, der dem Arbeitsbereich eines in der Führungsschiene geführten Fräsers entspricht. Dieser Arbeitssektor schneidet eine gedachte Verbindungslinie zwischen den Zangenköpfen, was gleichbedeutend damit ist, dass ein in der Führungsschiene aufgenommener Fräser zwischen den Zangenköpfen hindurchgeführt werden kann. Da die Zangenköpfe gerade den Facettengelenkfortsatz zwischen sich halten, ist die Bewegung des Fräsers durch die Führungsschiene genau auf den Bereich beschränkt, in dem der Facettengelenkfortsatz bearbeitet werden soll. Das Risiko, dass der Patient durch eine unbedachte Bewegung des Chirurgen verletzt wird, ist vermindert.

In einer vorteilhaften Ausführungsform ist die Führungsschiene um eine zweite Schwenkachse schwenkbar. Durch Schwenken um die zweite Schwenkachse kann die Ausrichtung des Arbeitssektors zwischen den Zangenköpfen verändert werden. Es kann also der Bereich, in dem der Fräser den Facettengelenkfortsatz bearbeitet, näher an den einen Zangenkopf oder an den anderen Zangenkopf angenähert werden. Dadurch ist es nicht mehr erforderlich, die Zangenköpfe absolut präzise zu positionieren. Leichte Ungenauigkeiten bei der Positionierung der Zangenköpfe können dadurch ausgeglichen werden, dass die Führungsschiene um die zweite Schwenkachse geschwenkt wird und damit der Arbeitssektor in die richtige Position zwischen den Zangenköpfen gebracht wird.

Die zweite Schwenkachse liegt vorzugsweise in der Ebene des Arbeitssektors. Dies hat den Vorteil, dass die Ausrichtung des Arbeitssektors sich nicht ändert, wenn die Führungsschiene um die zweite Schwenkachse geschwenkt wird.

Die Bewegungsfreiheit der Führungsschiene um die erste Schwenkachse ist normalerweise durch zwei Anschläge begrenzt. Durch die Anschläge sind zugleich die Grenzen des Arbeitssektors definiert. Das Gradmaß des Arbeitssektors, das dem Winkelbereich entspricht, den der Fräser überstreichen kann, ist vorzugsweise größer als 15°, weiter vorzugsweise größer als 30°, weiter vorzugsweise größer als 45°.

Für die Zangenbewegung der Zangenköpfe kann ein Zangengelenk vorgesehen sein, das zwei Zangenschenkel miteinander verbindet. Die Zangenschenkel können sich von den Zangenköpfen bis zu dem Zangengelenk und darüber hinaus bis zu einem Griffende des Führungsinstruments erstrecken. Das Griffende wird als proximales Ende des Führungsinstruments bezeichnet, das gegenüberliegende Ende, an dem die Zangenköpfe angeordnet sind, wird als distales Ende bezeichnet. In einer vorteilhaften Ausführungsform sind die zangenschenkel zwischen dem Zangengelenk und den Zangenköpfen aus einer senkrecht zu dem Zangengelenk ausgerichteten Ebene heraus abgewinkelt. Die Zangenköpfe liegen dadurch außerhalb der Ebene, die von den Griffenden der Zangenschenkel aufgespannt wird. Durch diese Gestaltung wird es erleichtert, um die nach hinten weisenden Dornfortsätze der Wirbelkörper herumzugreifen. Die Länge des abgewinkelten Teils der Zangenschenkel entspricht vorzugsweise ungefähr dem Dornfortsatz. Für die Bedienung des Führungsinstruments ist es außerdem günstig, wenn bezogen auf eine zur Achse des Zangengelenks senkrechte, sich durch das Zangengelenk erstreckende Ebene die Zangenköpfe auf der einen Seite und die erste Schwenkachse auf der anderen Seite angeordnet ist. Bezogen auf eine sich durch die Zangenköpfe erstreckende Längsachse ist vorzugsweise das Zangengelenk auf der einen Seite und die erste Schwenkachse auf der anderen Seite angeordnet.

An dem Zangenkopf kann eine Mehrzahl von Vorsprüngen ausgebildet sein, die in Richtung der Zangenbewegung ausgerichtet sind. Wenn also mit den Zangenköpfen an einem Facettengelenkfortsatz angegriffen wird, so kommen die Vorsprünge zuerst mit dem Facettengelenkfortsatz in Kontakt. Vorzugsweise laufen die Vorsprünge spitz zu, so dass sie leicht in das Knochengewebe eindringen können. Wenn die Vorsprünge in das Knochengewebe eindringen, bieten sie einen besseren Halt für das Führungsinstrument.

Beim Einsetzen einer Facettengelenkprothese werden regelmäßig sowohl die superiore oder als auch die inferiore Gelenkfläche ersetzt. Es muss dazu in beiden beteiligten Facettengelenkfortsätzen eine Ausnehmung zur Aufnahme des Schafts der Prothesenkomponente erzeugt werden. Mit dem erfindungsgemäßen Führungsinstrument wird die Möglichkeit eröffnet, die Ausnehmung in beiden Facettengelenkfortsätzen in einem einzelnen kombinierten Arbeitsschritt zu erzeugen. Dazu kann so mit dem Führungsinstrument an den Facettengelenkfortsätzen angegriffen werden, dass beide Facettengelenkfortsätze zwischen den Zangenköpfen fixiert sind. Mit einer Schwenkbewegung des Fräsers um die erste Schwenkachse kann dann eine Ausnehmung erzeugt werden, die sich durch beide Facettengelenkfortsätze erstreckt. Vorzugsweise sind die Vorsprünge dazu so am Zangenkopf angeordnet, dass in jeden der beiden Facettengelenkfortsätze mindestens ein Vorsprung eingreift. Dies kann dadurch erreicht werden, dass die Vorsprünge auf zwei Seiten einer sich durch den Zangenkopf erstreckenden Längsachse angeordnet sind.

Von der Erfindung umfasst ist außerdem ein Instrumentarium, das ein Führungsinstrument dieser Art sowie einen Fräser umfasst. Der Fräser zeichnet sich dadurch aus, dass er einen an die Führungsschiene angepassten Schaft aufweist. Der Schaft kann in die Führungsschiene eingelegt werden, so dass die Richtung des Fräsers definiert ist, wenn dieser sich in der Führungsschiene dreht. In Längsrichtung kann der Fräser gegenüber der Führungsschiene verschiebbar sein. Dadurch wird es möglich, unterschiedlich tiefe Ausnehmungen in den Facettengelenkfortsätzen zu erzeugen. Der Fräser ist mindestens so lang, dass er sich von der Führungsschiene bis zu der gedachten Verbindungslinie zwischen den Zangenköpfen erstreckt.

Beschrieben ist außerdem ein Verfahren zum Bearbeiten von Facettengelenkfortsätzen an Wirbelkörpern. Bei dem Verfahren wird zunächst eine schwenkbare Führungsschiene so relativ zu dem Facettengelenkfortsatz positioniert, dass ein mit der Schwenkbewegung der Führungsschiene überspannter Arbeitssektor den Facettengelenkfortsatz schneidet. Die Führungsschiene wird dann so relativ zu dem Facettengelenkfortsatz fixiert, dass die Führungsschiene auf Schwenkbewegungen beschränkt ist. Mit einem in die Führungsschiene eingesetzten Fräser wird der Facettengelenkfortsatz bearbeitet. Die Führungsschiene wird dazu zusammen mit dem Fräser so geschwenkt, dass der Fräser den Facettengelenkfortsatz bearbeitet und eine Ausnehmung erzeugt. Zum Durchführen des Verfahrens kann beispielsweise das erfindungsgemäße Führungsinstrument wie oben beschrieben verwendet werden.

Bei einer Ausführungsform des Verfahrens kann die Führungsschiene relativ zu zwei Facettengelenkfortsätzen fixiert werden. Die beiden Facettengelenkfortsätze, die vorzugsweise in einem Facettengelenk zusammenwirken, können dann in einem gemeinsamen Arbeitsschritt mit dem Fräser bearbeitet werden. Die Eindringtiefe des Fräsers in den Facettengelenkfortsatz kann durch Verschieben des Fräsers längs der Führungsschiene eingestellt werden. Weitere optionale Merkmale des Verfahrens ergeben sich aus der obigen Beschreibung der Bedienung des Führungsinstruments.

Die Erfindung wird nachfolgend unter Bezugnahme auf die beigefügten Zeichnungen anhand einer vorteilhaften Ausführungsform beispielhaft beschrieben. Es zeigen:
- Fig. 1:: einen Ausschnitt eines lumbalen Bereichs der Wirbelsäule;
- Fig. 2:: einen Ausschnitt zervikaler Wirbelkörper mit einer eingesetzten Facettengelenkprothese;
- Fig. 3:: einen Ausschnitt zervikaler Wirbelkörper mit einer anderen Ausführungsform einer Facettengelenkprothese;
- Fig. 4:: ein erfindungsgemäßes Führungsinstrument;
- Fig. 5:: eine andere Ansicht des Führungsinstruments aus Fig. 4;
- Fig. 6:: das distale Ende des Führungsinstruments in einer Fig. 5 entsprechenden Ansicht;
- Fig. 7:: einen vergrößerten Ausschnitt aus Fig. 6; und
- Fig. 8:: das distale Ende des Führungsinstruments in einer Fig. 4 entsprechenden Ansicht.

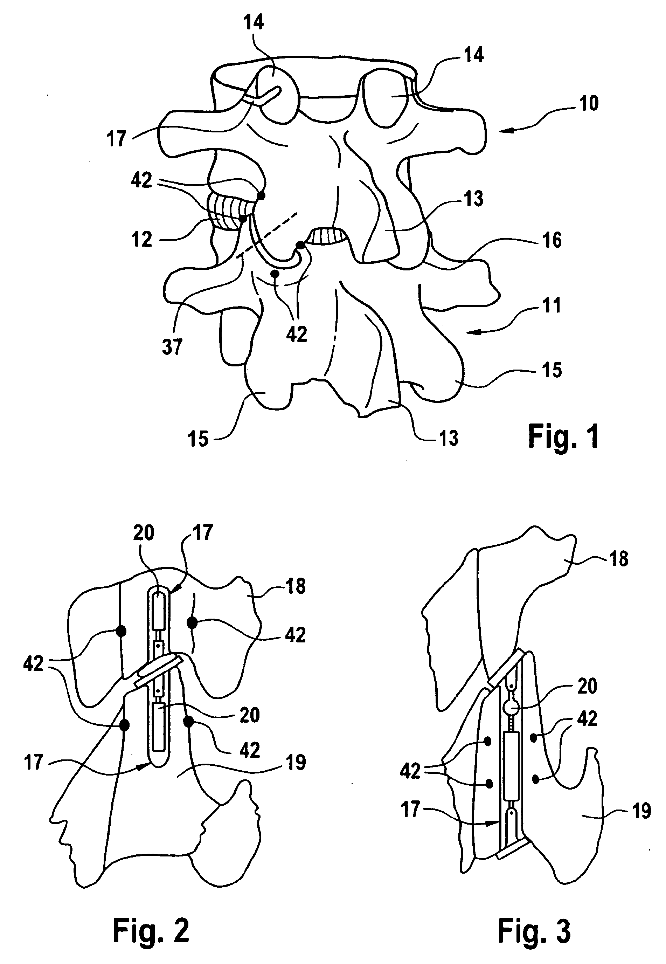

Ein in Fig. 1 dargestellter Ausschnitt einer menschlichen Wirbelsäule zeigt zwei lumbale Wirbelkörper 10, 11 mit einer dazwischenliegenden Bandscheibe 12 und jeweils einem nach hinten weisenden Dornfortsatz 13. Die Wirbelkörper 10, 11 umfassen jeweils zwei superiore Facettengelenkfortsätze 14 und zwei inferiore Facettengelenkfortsätze 15. Zwischen den beiden Wirbelkörpern 10, 11 wirken die inferioren Facettengelenkfortsätze des oberen Wirbelkörpers 10 und die superioren Facettengelenkfortsätze des unteren Wirbelkörpers 11 zusammen und bilden zwei Facettengelenke 16. Bei einer Bewegung der Wirbelkörper vollführen die Gelenkflächen der Facettengelenke 16 eine Gleitbewegung relativ zueinander.

Mit einer in Fig. 1 nicht dargestellten Facettengelenkprothese werden die Gelenkflächen der Facettengelenke 16 durch künstliche Gelenkflächen ersetzt. Zur Befestigung der Prothese erstreckt sich ein Schaft durch den Facettengelenkfortsatz, der von der Gegenseite gespannt wird. Für die Aufnahme des Schafts werden Ausnehmungen in den Facettengelenkfortsätzen erzeugt. Eine solche Ausnehmung 17 ist beispielhaft für den superioren Facettengelenkfortsatz 14 des oberen Wirbelkörpers 10 angedeutet.

Entsprechende Facettengelenkprothesen können auch im zervikalen Bereich der Wirbelsäule verwendet werden. Die Figuren 2 und 3 zeigen jeweils Ausschnitte der inferioren Facettengelenkfortsätze 18 und der superioren Facettengelenkfortsätze 19 zweier benachbarter zervikaler Wirbelkörper. In den Facettengelenkfortsätzen 18, 19 sind Ausnehmungen 17 erzeugt, die die Schäfte von verschiedenen Ausführungsformen von Facettengelenkprothesen 20 aufnehmen.

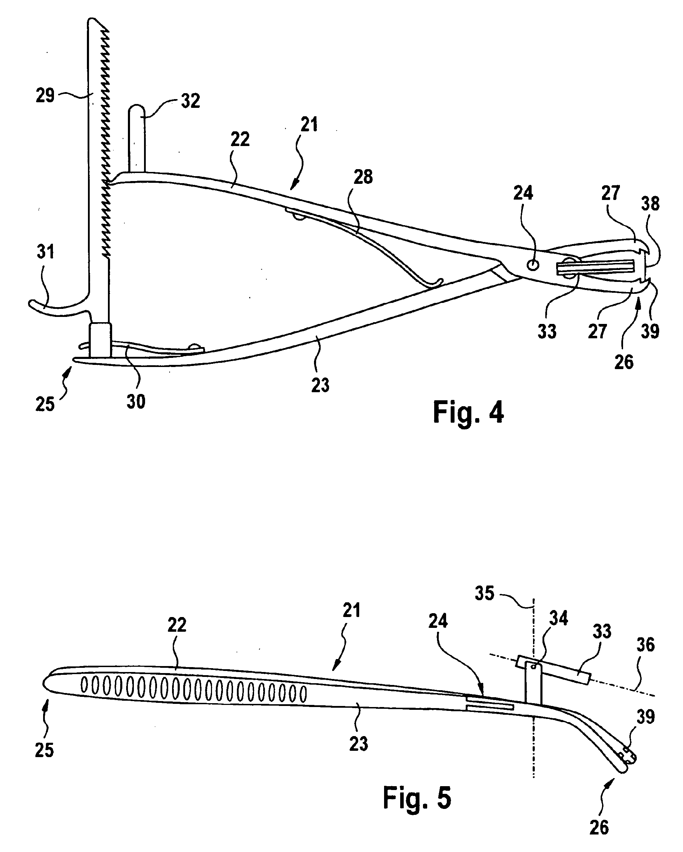

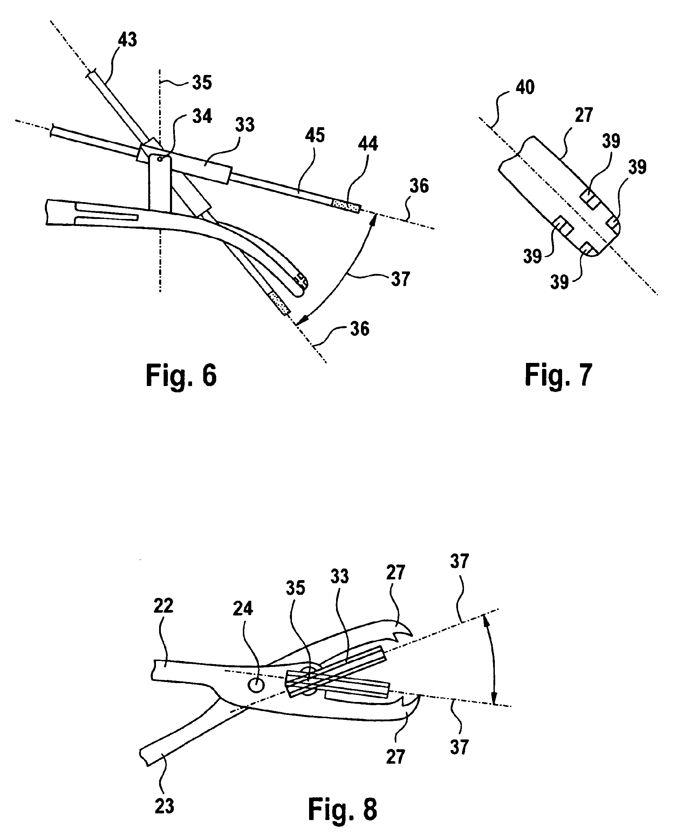

Das erfindungsgemäße chirurgische Instrumentarium aus einem Führungsinstrument 21 und einem Fräser 45 dient dazu, die Ausnehmungen 17 in den Facettengelenkfortsätzen 14, 15, 18, 19 zu erzeugen. Das in den Figuren 4 und 5 dargestellte Führungsinstrument 21 umfasst zwei Zangenschenkel 22, 23, die über ein Zangengelenk 24 miteinander verbunden sind. Die Zangenschenkel 22, 23 erstrecken sich von einem proximalen Ende 25 des Führungsinstruments 21 bis zu einem distalen Ende 26. Am distalen Ende 26 sind Zangenköpfe 27 ausgebildet, mit denen das Führungsinstrument 21 an den Facettengelenkfortsätzen 14, 15, 18, 19 angreift. Jeder der Zangenköpfe 27 umfasst gemäß Fig. 7 auf seiner Innenseite vier Vorsprünge 39, die in Richtung des jeweils gegenüberliegenden Zangenkopfs 27 und damit in Richtung der Zangenbewegung ausgerichtet sind. Die Vorsprünge 39 sind zu zwei Seiten einer sich durch den Zangenkopf 27 erstreckenden Längsachse 40 angeordnet. Wenn mit den Zangenköpfen 27 an den Facettengelenkfortsätzen 14, 15, 18, 19 angegriffen wird, dringen die Vorsprünge 39 in die Facettengelenkfortsätze 14, 15, 18, 19 ein. Durch die spitz zulaufende Form der Vorsprünge 39 wird das Eindringen erleichtert.

Vom proximalen Ende 25 aus bedient der Chirurg das Führungsinstrument 21. Insbesondere drückt der Chirurg die proximalen Ende der Zangenschenkel 22, 23 entgegen der Kraft einer Feder 28 zusammen, um mit den Zangenköpfen 27 an den Facettengelenkfortsätzen 14, 15, 18, 19 anzugreifen. Durch ein Rastelement 29 wird das Führungsinstrument 21 in gespanntem Zustand gehalten. Eine an dem Zangenschenkel 23 ausgebildete Feder 30 hält das Rastelement 29 in Eingriff mit dem gegenüberliegenden Zangenschenkel 22. Ein Griffelement 31 an dem Rastelement 29 dient dazu, das Rastelement 29 aus dem Eingriff zu lösen. Durch einen Vorsprung 32 an dem Zangenschenkel 22 wird verhindert, dass der Chirurg mit der Zahnung des Rastelements 29 in Berührung kommt.

Zwischen dem Zangengelenk 24 und den Zangenköpfen 27 ist eine Führungsschiene 33 angeordnet, die eine Führungsachse 36 definiert. Die Führungsschiene 33 ist um eine erste Schwenkachse 34 und um eine zweite Schwenkachse 35 relativ zu den Zangenschenkeln 22, 23 schwenkbar. Die erste Schwenkachse 34 ist senkrecht zur Führungsachse 36 ausgerichtet. Die zweite Schwenkachse 35 ist im Wesentlichen parallel zur Achse des Zangengelenks 24 ausgerichtet.

Der Fräser 45 des erfindungsgemäßen Instrumentariums umfasst einen Schaft 43 und einen Arbeitskopf 44. Die Führungsschiene 33 ist gemäß Fig. 6 so ausgebildet, dass sie den Schaft 43 des Fräsers 45 aufnehmen kann. Die Achse des Fräsers 45 wird durch die Führungsschiene 33 auf die Führungsachse 36 festgelegt. Wenn der Fräser 45 sich in der Führungsschiene 33 dreht, entspricht die Drehachse der Führungsachse 36. Wird die Führungsschiene 33 um die erste Schwenkachse 34 geschwenkt, überstreicht die Führungsachse 36 einen Arbeitssektor 37. Der Arbeitssektor 37 schneidet eine gedachte Verbindungslinie 38 zwischen den Zangenköpfen 27. Durch Schwenken der Führungsschiene 33 kann der Arbeitskopf 44 des Fräsers 45 also zwischen den Zangenköpfen 27 hindurch bewegt werden. Durch Schwenken der Führungsschiene 33 um die zweite Schwenkachse 35 kann gemäß Fig. 8 die Ausrichtung des Arbeitssektors 37 zwischen den Zangenköpfen 27 geändert werden. In Längsrichtung ist der Fräser 45 relativ zu der Führungsschiene 33 beweglich.

Um eine Ausnehmung 17 in den Facettengelenkfortsätzen 14, 15, 18, 19 zu erzeugen, positioniert der Chirurg das Führungsinstrument 21 an einem Facettengelenkfortsatz und drückt die proximalen Enden des Führungsinstruments 21 zusammen, so dass die Zangenköpfe 27 den Facettengelenkfortsatz zwischen sich einklemmen. Die Vorsprünge 39 dringen dabei in das Knochengewebe des Facettengelenkfortsatzes ein. Die ungefähren Punkte des Eindringens sind in den Figuren 1 bis 3 mit dem Bezugszeichen 42 angedeutet. Die Längsachse 40 des Zangenkopfs 27 erstreckt sich jeweils zwischen zwei benachbarten Punkten 42. In den Figuren 1 und 2 werden mit dem Einklemmen der Facettengelenkfortsätze 14, 15, 18, 19 zwischen den Zangenköpfen 27 zugleich die Facettengelenkfortsätze 14, 15 bzw. 18, 19 in eine definierte Position zueinander gebracht.

Wenn die Führungsschiene 33 auf diese Weise in eine definierte Position gebracht ist und auf Schwenkbewegungen relativ zu den Facettengelenkfortsätzen 14, 15, 18, 19 beschränkt ist, setzt der Chirurg den Fräser 45 in die Führungsschiene 33 ein. Durch Schwenken um die zweite Schwenkachse 35 wird der Arbeitskopf 44 des Fräsers 45 so positioniert, dass er die richtige Stellung zwischen den Zangenköpfen 27 hat. Bei dem anschließenden eigentlichen Bearbeitungsschritt wird die Führungsschiene 33 um die erste Schwenkachse 34 geschwenkt und der Arbeitskopf 44 des Fräsers 45 trägt während dieser Schwenkbewegung Material von den Facettengelenkfortsätzen ab, um die Ausnehmungen 17 zu erzeugen. Durch Längsverschiebung des Fräsers 45 in der Führungsschiene 33 wird bestimmt, wie tief der Arbeitskopf 44 des Fräsers 45 in den Facettengelenkfortsatz eingreift. In Fig. 1 ist der Arbeitssektor 37, in dem sich der Arbeitskopf 44 des Fräsers 45 während der Bearbeitung bewegt, durch eine gestrichelte Linie angedeutet. In den Figuren 1 und 2 werden mit einem einzelnen Bearbeitungsschritt Ausnehmungen 17 gleichzeitig in zwei Facettengelenkfortsätzen 14, 15 bzw. 18, 19 erzeugt. In die Ausnehmungen 17 kann eine Facettengelenkprothese 20 wie in den Figuren 2 und 3 gezeigt eingesetzt werden.

## Patentansprüche

1. Chirurgisches Führungsinstrument zur Verwendung an Facettengelenkfortsätzen (14, 15, 18, 19) von Wirbelkörpern (10, 11), an dessen distalen Ende (26) zwei Zangenköpfe (27) ausgebildet sind, die für eine Zangenbewegung relativ zueinander ausgelegt sind, und bei dem eine Führungsschiene (33) vorgesehen ist, die eine Führungsachse (36) für einen Fräser (45) definiert, wobei die Führungsschiene (33) um eine erste Schwenkachse (34) schwenkbar ist, so dass die Führungsachse (36) mit der Schwenkbewegung um die erste Schwenkachse (34) einen Arbeitssektor (37) überspannt, **dadurch gekennzeichnet, dass** der Arbeitssektor (37) eine gedachte Verbindungslinie (38) zwischen den Zangenköpfen (27) schneidet.

2. Führungsinstrument nach Anspruch 1, **dadurch gekennzeichnet, dass** die Führungsschiene (33) um eine zweite Schwenkachse (35) schwenkbar ist.

3. Führungsinstrument nach Anspruch 2, **dadurch gekennzeichnet, dass** die zweite Schwenkachse (35) in der Ebene des Arbeitssektors (37) liegt.

4. Führungsinstrument nach einem der Ansprüche 1 bis 3, **dadurch gekennzeichnet, dass** das Gradmaß des Arbeitssektors (37) größer ist als 15°, vorzugsweise größer als 30°, weiter vorzugsweise größer als 45°.

5. Führungsinstrument nach einem der Ansprüche 1 bis 4, **dadurch gekennzeichnet, dass** Zangenschenkel (22, 23) vorgesehen sind, die sich zwischen den Zangenköpfen (27) und einem die Zangenschenkel (22, 23) verbindenden Zangengelenk (24) erstrecken und dass die Zangenschenkel (22, 23) aus einer senkrecht zu dem Zangengelenk (24) ausgerichteten Ebene heraus abgewinkelt sind.

6. Führungsinstrument nach einem der Ansprüche 1 bis 5, **dadurch gekennzeichnet, dass** bezogen auf eine zum Zangengelenk (24) senkrechte, sich durch das Zangengelenk (24) erstreckende Ebene die Zangenköpfe (27) auf der einen Seite und die erste Schwenkachse (34) auf der anderen Seite angeordnet sind.

7. Führungsinstrument nach einem der Ansprüche 1 bis 6, **dadurch gekennzeichnet, dass** eine Mehrzahl von Vorsprüngen (39) für einen Zangenkopf (27) vorgesehen ist, dass die Vorsprünge (39) in Richtung der Zangenbewegung ausgerichtet sind und dass die Vorsprünge (39) zu zwei Seiten einer sich durch den Zangenkopf (27) erstreckenden Längsachse (40) angeordnet sind.

8. Instrumentarium zum Bearbeiten von Facettengelenkfortsätzen (14, 15, 18, 19) an Wirbelkörpern (10, 11), umfassend ein Führungsinstrument (21) nach einem der Ansprüche 1 bis 7 und einen Fräser (45), wobei der Fräser (45) einen an die Führungsschiene (33) angepassten Schaft (43) aufweist.

9. Instrumentarium nach Anspruch 8, **dadurch gekennzeichnet, dass** der Schaft (43) des Fräsers (45) in Längsrichtung in der Führungsschiene (33) verschiebbar ist.

## Claims

1. Surgical guide instrument for use on the articular processes (14, 15, 18, 19) of vertebral bodies (10, 11), at the distal end (26) of which guide instrument there are two plier heads (27), which are designed for a plier movement relative to each other, and in which a guide rail (33) is provided, which defines a guide axis (36) for a reamer (45), wherein the guide rail (33) is pivotable about a first pivot axis (34), such that the guide axis (36), with the pivoting movement about the first pivot axis (34), spans a work sector (37), **characterized in that** the work sector (37) intersects an imaginary connecting line (38) between the plier heads (27).

2. Guide instrument according to Claim 1, **characterized in that** the guide rail (33) is pivotable about a second pivot axis (35).

3. Guide instrument according to Claim 2, **characterized in that** the second pivot axis (35) lies in the plane of the work sector (37).

4. Guide instrument according to one of Claims 1 to 3, **characterized in that** the extent of the work sector (37) in degrees is greater than 15°, preferably greater than 30°, more preferably greater than 45°.

5. Guide instrument according to one of Claims 1 to 4, **characterized in that** plier limbs (22, 23) are provided, which extend between the plier heads (27) and a plier joint (24) connecting the plier limbs (22, 23), and **in that** the plier limbs (22, 23) are angled out from a plane oriented perpendicular to the plier joint (24).

6. Guide instrument according to one of Claims 1 to 5, **characterized in that**, in relation to a plane lying perpendicular to the plier joint (24) and extending through the plier joint (24), the plier heads (27) are arranged on one side and the first pivot axis (34) on the other side.

7. Guide instrument according to one of Claims 1 to 6, **characterized in that** a plurality of projections (39) are provided for a plier head (27), that the projections (39) are oriented in the direction of the plier movement, and that the projections (39) are arranged on both sides of a longitudinal axis (40) extending through the plier head (27).

8. Instrument set for working the articular processes (14, 15, 18, 19) of vertebral bodies (10, 11), comprising a guide instrument (21) according to one of Claims 1 to 7, and a reamer (45), said reamer (45) having a shaft (43) adapted to the guide rail (33).

9. Instrument set according to Claim 8, **characterized in that** the shaft (43) of the reamer (45) is movable in the longitudinal direction in the guide rail (33).

## Revendications

1. Instrument de guidage chirurgical destiné à être utilisé sur des appendices (14, 15, 18, 19) de facettes d'articulation de corps de vertèbres (10, 11), deux têtes de pince (27) conçues pour permettre un déplacement relatif mutuel étant formées à l'extrémité distale (26) de l'instrument, un rail de guidage (33) qui définit un axe de guidage (36) pour une fraise (45) étant prévu, le rail de guidage (33) pouvant pivoter autour d'un premier axe de pivotement (34) de telle sorte que l'axe de guidage (36) s'étende sur un secteur de travail (37) qui entoure le premier axe de pivotement (34) lors du déplacement de pivotement, **caractérisé en ce que**
le secteur de travail (37) coupe une ligne idéale de liaison (38) entre les têtes (27) de pince.

2. Instrument de guidage selon la revendication 1, **caractérisé en ce que** le rail de guidage (33) peut pivoter autour d'un deuxième axe de pivotement (35).

3. Instrument de guidage selon la revendication 2, **caractérisé en ce que** le deuxième axe de pivotement (35) est situé dans le plan du secteur de travail (37).

4. Instrument de guidage selon l'une des revendications 1 à 3, **caractérisé en ce que** la dimension angulaire du secteur de travail (37) est supérieure à 15°, de préférence supérieure à 30° et de façon encore plus préférable supérieure à 45°.

5. Instrument de guidage selon l'une des revendications 1 à 4, **caractérisé en ce que** les branches de pince (22, 23) prévues s'étendent entre les têtes de pince (27) et une articulation de pince (24) qui relie les branches de pince (22, 23) et **en ce que** les branches de pince (22, 23) sont coudées hors d'un plan orienté perpendiculairement à l'articulation de pince (24).

6. Instrument de guidage selon l'une des revendications 1 à 5, **caractérisé en ce que** les têtes de pince (27) sont disposées sur le premier côté et le premier axe de pivotement (34) sur l'autre côté d'un premier plan perpendiculaire à l'articulation de pince (24) et qui s'étend à travers l'articulation de pince (24).

7. Instrument de guidage selon l'une des revendications 1 à 6, **caractérisé en ce que** plusieurs saillies (39) pour une tête de pince (27) sont prévues, **en ce que** les saillies (39) sont orientées dans la direction du déplacement des pinces et **en ce que** les saillies (39) sont disposées sur deux côtés d'un axe longitudinal (40) qui s'étend à travers la tête de pince (27).

8. Trousse d'instruments destinée à traiter des appendices (14, 15, 18, 19) de facettes d'articulation de corps de vertèbres (10, 11) et comprenant un instrument de guidage (21) selon l'une des revendications 1 à 7 et une fraise (45), la fraise (45) présentant une tige (43) adaptée au rail de guidage (33).

9. Trousse d'instruments selon la revendication 8, **caractérisée en ce que** la tige (43) de la fraise (45) peut coulisser dans le sens de la longueur dans le rail de guidage (33).
